(19) Europäisches Patentamt

European Patent Office

Office européen des brevets

(11) **EP 0 663 239 B1**

(12) **EUROPÄISCHE PATENTSCHRIFT**

(45) Veröffentlichungstag und Bekanntmachung des Hinweises auf die Patenterteilung:
**30.07.1997 Patentblatt 1997/31**

(51) Int. Cl.[6]: **B01L 3/00**, G01N 33/18,
B01L 3/14, G01N 21/78

(21) Anmeldenummer: 95100318.5

(22) Anmeldetag: **11.01.1995**

(54) **Vorrichtung zur chemischen Analyse von Probeninhaltsstoffen**

Device for the chemical analysis of sample components

Dispositif pour l'analyse chimique des composants d'un échantillon

(84) Benannte Vertragsstaaten:
**AT DE FR IT NL**

(30) Priorität: **12.01.1994 DE 4400630**

(43) Veröffentlichungstag der Anmeldung:
**19.07.1995 Patentblatt 1995/29**

(73) Patentinhaber: **Dr. Bruno Lange GmbH**
**D-40549 Düsseldorf (DE)**

(72) Erfinder:
• **Farjam, Aria**
**D-41069 Mönchengladbach (DE)**

• **Kraatz, Christian**
**D-40670 Meerbusch (DE)**
• **Ketterer, Klaus**
**D-40667 Meerbusch (DE)**

(74) Vertreter: **Fitzner, Uwe, Dr. et al**
**Dres. Fitzner & Christophersen,**
**Rechts- und Patentanwälte,**
**Kaiserswerther Str. 74**
**40878 Ratingen (DE)**

(56) Entgegenhaltungen:
US-A- 3 741 727          US-A- 4 201 548
US-A- 4 315 890          US-A- 5 094 955
US-A- 5 275 956          US-A- 5 320 807

**Beschreibung**

Die vorliegende Erfindung betrifft ein Testkit zur chemischen Analyse von gasförmigen oder in die Gasform überführbaren Probeninhaltsstoffen.

In der analytischen Chemie sind solche Methoden von besonderer Bedeutung, bei denen der zu bestimmende Parameter durch Überführung in die Gasform selektiv aus dem Probengemisch abgetrennt wird. Hierbei kann nach Überführung in die Gasphase eine direkte Bestimmung mittels Gaschromatographie, Atomabsorption, oder IR- und Chemoluminiszenzspektrometrie durchgeführt werden. Daneben besteht auch die Möglichkeit einer indirekten Bestimmung mit den Methoden der Konduktometrie, der Coulometrie, der Potentiometrie, der Gasvolumetrie, der acidimetrischen Maßanalyse, der manometrischen Messung, der jodometrischen Maßanalyse und der Photometrie.

Die US-A-5,320,807 beschreibt eine Testvorrichtung, mit der der Zustand und Prozeßverlauf in Kompostanlagen überwacht werden kann. Zu diesem Zweck wird eine Probe des Kompostes in ein geschlossenes Gefäß gebracht und dort für einen Zeitraum von einigen Stunden in Ruhe gelassen. Während dieses Zeitraums stellt sich in Gasform des Containers ein Gleichgewicht ein, das durch Austreten von $CO_2$ und flüchtigen organischen Säuren aus dem Kompost bestimmt wird. Mit einem oder mehreren Nachweisreagenzien, die in dem Gasraum des Containers aufgehängt sind, ist es sodann möglich, die Konzentration von $CO_2$ oder den organischen flüchtigen Säuren durch die optische Veränderung der Reagenzien nachzuweisen und zu messen. Die gesamte Vorrichtung wird nur für die Untersuchung von Kompostproben eingesetzt. Nachteilig an ihr ist, daß sie keine quantitativen Messungen erlaubt und bei ihrer Befüllung sowohl der Proben- als auch der Nachweisraum zu öffnen sind.

Die US-A-4,315,890 beschreibt eine Vorrichtung, mit der flüchtige Probenbestandteile, insbesondere aus Körperflüssigkeiten bestimmt werden sollen. Es erfolgt somit keine Erzeugung von Gasen, sondern lediglich die Austreibung vorhandener Gase. Der Nachweis der ausgetriebenen Gase erfolgt in einem geschlossenen Gefäß durch Reaktion mit bzw. Absorption an speziellen, räumlich getrennt angeordneten, gassensitiven Nachweisreagenzien. Die Vorrichtung besteht dabei aus zwei ineinanderschiebbaren Glastuben (nach Art einer Spritze). Auch hierbei ist nachteilig, daß die Vorrichtung während des Befüllens insgesamt offensteht. Eine Verbindung nach außen muß ferner bestehen, um die Gefäßteile ineinander schieben zu können und dabei das verdrängte Volumen auszugleichen.

Bedeutung haben die genannten Methoden vor allen Dingen für die Wasseranalytik (vgl. allgemein: „Deutsche Einheitsverfahren zur Wasser-, Abwasser- und Schlamm-Untersuchung" (DEV), 28 Lieferung, Verlag Chemie; „Standard Methods - For the Examination of Water and Wastewater", 16. Aufl., American Public Health Association, Washington DC). Hier können mit den Methoden der IR-Spektrometrie u.a. Sulfite, Carbonate, Chloride, Sulfide, Cyanide, Ammonium, organisch gebundener Kohlenstoff, gebundener Stickstoff und Nitrate bestimmt werden, nachdem die betreffenden Komponenten in ein gasförmiges Produkt, d.h. in Schwefeldioxid, Kohlendioxid, Chlorwasserstoff, Schwefelwasserstoff, Cyanwasserstoff, Ammoniak und Stickoxide überführt worden sind. Als Umsetzungsmethode für die Überführung in die Gasphase dienen vor allem das Ansäuern, das Alkalisieren, die Oxidation und die Reduktion der Probe.

Für andere Komponenten eignet sich nach Überführung in ein gasförmiges Produkt die Atomabsorption. So lassen sich beispielsweise Quecksilber, Arsen, Selen, Bismut, Antimon, Zinn und Tellur durch Reduktion in gasförmige Produkte überführen, die sich direkt für die Atomabsorption eignen.

Nach dem Gesagten ist zu erkennen, daß im Regelfall eine Überführung der zu bestimmenden Probenbestandteile in die Gasphase erforderlich ist. Sofern mit indirekten Bestimmungsmethoden gearbeitet wird, muß der Gasanteil in flüssige Vorlagen überführt werden. Ein vollständiger Analysengang gliedert sich demgemäß in folgende Teilschritte:

1. Chemische Umsetzung der Probe zur Bildung gasförmiger oder gasförmig austreibbarer Produkte

2. Austreibung des Gases durch inertes Trägergas (Strippen), z.B. durch Wasserdampfdestillation (Trägergas = Wasserdampf) oder durch überschüssiges Reaktionsgas (z.B. bei der oxidativen Pyrolyse).

3. Transport des Gases in ein Absorptionsgefäß (meistens durch den Trägergasstrom über eine Schlauch- oder Rohrverbindung)

4. Absorption in der flüssigen Phase oder auch an einer Festphase.

5. Analyse der Absorptionsvorlage.

Es sind Geräte entwickelt worden, welche alle genannten Teilschritte automatisiert durchführen können. Diese Geräte sind vor allem wegen des erforderlichen Gastransports apparativ relativ aufwendig. Hinzu kommt, daß diese Systeme regelmäßig nur für einen speziellen Anwendungsfall, ausnahmsweise auch für einen zweiten oder dritten Anwendungsfall, geeignet sind. Die Kosten solcher Apparaturen liegen regelmäßig bei mehreren zehntausend DM. Nachteilig ist außerdem, daß die bisher bekannten Apparaturen eine hohe fachliche Qualifikation des Personals erfordern. Aus diesen Gründen haben sich die bisher am Markt erhältlichen Geräte im betriebsanalytischen Labor, d.h. in Bereichen mit beschränkter Laborausstattung und analytisch nicht ausreichend geschultem Personal nicht durchgesetzt.

Nach dem Regelwerk der Abwassertechnischen Vereinigung (ATV), September 1991, Hinweis H 704, Analysenverfahren zur Eigenüberwachung von Abwasserbehandlungsanlagen durch Betriebspersonal, wird verlangt, daß die Verfahren

1. einfach und schnell vor Ort (z.B. direkt an den Kläranlagen), durch chemisch nicht oder wenig vorgebildetes Personal durchzuführen sein sollen,

2. in der Anschaffung sowie im betrieblichen Einsatz wirtschaftlich sind, und

3. sich in Anwendung und Entsorgung umweltverträglich verhalten.

Diese wesentlichen Anforderungen der ATV werden von den geschilderten Apparaturen nicht erfüllt. In der Konsequenz finden daher die genannten Apparaturen nur Anwendung bei Überwachungsbehörden und größeren Laboratorien, nicht jedoch bei der großen Mehrzahl der zur Eigenüberwachung verpflichteten kommunalen Kläranlagen-Betreiber und industriellen Indirekteinleiter.

Dem Betriebspersonal solcher Anlagen steht nach dem Gesagten regelmäßig nur die Minimalausstattung eines Labors zur Verfügung. Diese besteht neben den laborüblichen Glas-, Kunststoff- und Dosiergeräten üblicherweise aus einem universellen und somit auch modular aufgebauten Analysensystem, welches sich im Prinzip aus zwei Komponenten zusammensetzt, nämlich einer Basiskomponente und einer variablen Komponente.

Die unveränderliche Basiskomponente besteht in der Regel aus einem Photometer und einem Heizblock-Trockenthermostat.

Bei den variablen Komponenten handelt es sich um chemische Testkits, die alle zur Analyse benötigten Verbrauchsmaterialien - dies sind im wesentlichen die benötigten Reagenzien - enthalten. Die Variabilität besteht in der Verfügbarkeit einer großen Zahl unterschiedlicher Testkits zur Bestimmung diverser, chemischer Probeninhaltsstoffe, wobei jedes einzelne Testkit nur die Quantifizierung einer speziellen Substanz oder Substanzgruppe (sog. "Summenparameter") in der Probe ermöglicht.

Ein solches modulares Analysensystem erlaubt es, unter Verwendung der immer gleichen Gerätebausteine (Photometer, Trockenthermostat) verschiedene Analysenparameter in Verbindung mit den hierfür ausgelegten preiswerten Testkits zu bestimmen. Eine betriebsanalytische Laborausstattung ist in diesem Sinne modular aufgebaut, so daß bei geringen Einzelkosten der Geräte und der Testkits die Einzelkomponenten des modularen Analysensystems für die jeweilige Aufgabenstellung kombinierbar und für unterschiedliche Arbeiten mehrfach nutzbar sind.

Aufgabe der vorliegenden Erfindung ist es nach dem Gesagten, einen Testkit für die Bestimmung von gasförmigen oder in die Gasform überführbaren Probeninhaltsstoffen zur Verfügung zu stellen, der vorzugsweise mit den am Markt befindlichen universellen Analysensystemen eingesetzt werden kann, insbesondere solchen, die ein Photometer (als Auswerteeinheit) in Kombination mit einem Trockenthermostaten nutzen. Der Testkit ist jedoch auch für andere Auswerteverfahren, wie z.B. reflektometrische, fluorimetrische, luminometrische oder elektrochemische Meßverfahren (Potentiometrie, Coulometrie, Konduktometrie, u.a.) einsetzbar.

Die erfindungsgemäße Aufgabe wird durch einen Testkit gelöst,

a) mit zwei separaten einzelnen Behältern, von denen der erste der Aufnahme der Probe und der zweite der Aufnahme der aus der Probe freigesetzten Gase dient,

wobei

b) der zweite Behälter ein gassensitives Reagenz enthält, das durch Kontakt mit dem in dem ersten Behälter erzeugten Gas eine optische Änderung erfährt,

c) der zweite Behälter so ausgestaltet ist, daß er in ein optisches Meßgerät als Meßvorlage einsetzbar ist,

d) die Behälter jeweils mit einem Verschluß versehen sind, und

e) der Testkit weiterhin einen Adapter umfaßt, durch den die Behälter nach Abnahme der Verschlüsse gasdicht miteinander verbindbar sind.

Der Testkit bildet somit ein verschließbares Behältnis (aus zwei Behältern und dem Adapter gebildet), in dem innerhalb eines örtlich abgegrenzten Bereiches eine Reaktionszone angeordnet ist, die zur Aufnahme einer Probe und zur Gaserzeugung aus dieser Probe dient, und in dem in einem weiteren abgegrenzten Bereich eine Detektionszone mit einem gassensitiven Reagenz angeordnet ist, die zur Detektion der in der Reaktionszone erzeugten Gase dient, wobei die Reaktionszone mit der Detektionszone nur über den Gasraum chemisch verbunden ist.

Das Behältnis des Testkits besteht aus zwei separaten einzelnen Behältern, die mittels eines Adapters miteinander verbindbar sind. Einer der Behälter dient dabei zur Aufnahme der Probe und enthält die Reaktionszone, während der

EP 0 663 239 B1

andere Behälter der Aufnahme des festen oder flüssigen, gassensitiven Reagenzes dient, und die Detektionszone enthält. Die Koppelung durch den Adapter verbindet die beiden Behälter und schließt sie gleichzeitig gasdicht gegen den Außenraum ab.

Der Adapter kann dabei so ausgestaltet sein, daß er eine selektive semipermeable Membran enthält, die die Innenräume der Behälter voneinander trennt. Vorzugsweise besteht die Selektivität der Membran in einer ausschließlichen Durchlässigkeit für Gase. Die Membran kann erfindungsgemäß aus einem hydrophoben Material bestehen.

Die Anwendung des Testkits mit dem Adapter und der integrierten Membran kann dermaßen aussehen, daß zuerst der Behälter mit der Detektionszone, der zwecks Lagerstabilität mit einem geeigneten Verschluß versehen ist, und eine vorkonfektionierte Indikatorlösung oder ein anderes geeignetes Nachweisreagenz enthält, geöffnet wird, und anstelle des Verschlusses der Adapter mit der integrierten Membran aufgeschraubt wird. Ebenso wird der zweite Behälter, der die Reaktionszone enthält, geöffnet. Auch er kann die zur Analyse notwendigen Stoffe in vorkonfektionierter Form enthalten. Anschließend wird die zu analysierende Probe in den Behälter mit der Reaktionszone gegeben, und beide Behälter werden mit Hilfe des Adapters gasdicht gegen den Außenraum miteinander verbunden. Ggf. ist daraufhin der Behälter mit der Reaktionszone geeignet zu behandeln, z.B. durch Erwärmung, um die Erzeugung und Freisetzung der nachzuweisenden Gase zu fördern bzw. den Gastransfer in die Detektionszone zu beschleunigen. Nach der Freisetzung der Gase und ihrer Reaktion mit dem Nachweisreagenz in der Detektionszone werden die so erzeugten Veränderungen mit geeigneten Meßmethoden nachgewiesen.

Eine weitere besondere Ausgestaltung des Adapters besteht darin, daß er die oberen Bereiche der Behälter, d.h. deren Gasraum - ohne eine Trennung der Innenräume durch eine semipermeable Membran - miteinander verbindet. Hierdurch wird erreicht, daß nur der Austausch bzw. Transfer von Gasen zwischen den Behältern möglich ist.

Es ist auch möglich, daß der Adapter einen geschlossenen Gaskreislauf zwischen den Behältern herstellt, wobei die Geschwindigkeit und Richtung des Gastransports durch eine in den Adapter integrierte Gaspumpe gesteuert werden kann.

Vorzugsweise sieht der geschlossene Gaskreislauf derart aus, daß die jeweiligen Gaszuführungen durch die Reaktions- bzw. Detektionszone geleitet werden, während die Gasausgänge sich im Gasraum der Behälter befinden. Diese Anordnung ist insbesondere dann sinnvoll, wenn in der Detektions- und/oder der Reaktionszone flüssige Phasen vorhanden sind. In diesem Falle wird nämlich durch den eingeleiteten Gasstrom eine starke Durchmischung der Flüssigkeiten und ein enger Kontakt mit dem Gas erreicht. Auf diese Weise kann nach Art eines Stripprozesses die Freisetzung der nachzuweisenden Gase beschleunigt bzw. deren Umsetzung in der Detektionszone gefördert werden.

Für das feste oder flüssige gassensitive Reagenz kommt beispielsweise eine optisch sensitive Festphasendetektionsschicht, vorzugsweise eine Optodenmembran, zur Anwendung, die in der Detektionszone angeordnet wird. Bei Optodenmembranen handelt es sich um filmartige Schichten auf Polymerbasis, die auf chemische Beeinflussung, z.B. durch die zu analysierenden Gase, mit einer Änderung ihres optischen Verhaltens, beispielsweise einer Farbänderung, reagieren. Solche Optodenmembranen, in diesem Fall bestehend aus plastifizierter Ethylcellulose und einem inkorporierten pH-Indikator mit selektiver Ansprache auf Kohlendioxid, wurden z.B. von A. Mills und Mitarbeitern in Anal. Chem.1992, 64, 1383-1389 beschrieben. In ähnlicher Weise reagieren von M. Kuratli und Mitarbeitern (Anal.Chem.1993, 65, 3473-3479) entwickelte Optodenmembranen auf Basis von plastifiziertem PVC und einem inkorporierten lipophilen Benzaldehydderivat mit einer Änderung ihrer UV-Absorption ($\lambda_{max}$ = 256 nm), sobald sie mit Schwefeldioxid in Kontakt gebracht werden.

Eine entsprechende Ausgestaltung des Behältnisses vorausgesetzt, kann das gassensitive Reagenz auch eine Flüssigkeit sein, die vorzugsweise einen gelösten Indikator oder ein Farbreagenz enthält.

Für eine Integration der erfindungsgemäßen Vorrichtung in ein marktübliches Analysensystem ist diese so ausgestaltet, daß sie in ein optisches Meßgerät als Meßvorlage eingesetzt werden kann. Dabei kann es sich insbesondere um die Ausgestaltung als Küvette für ein Photometer handeln.

In Verbindung mit allen vorgenannten Varianten des erfindungsgemäßen Testkits kann dieser für spezielle Anwendungsfälle so modifiziert werden, daß er in mindestens einem Teilbereich (Reaktions- oder Detektionszone) in geeigneter Weise räumlich unterteilt ist, so daß in diesem Teilbereich zunächst voineinander separiert gehaltene Proben, Reagenzien oder Phasen erst durch einfache mechanische Manipulationen, wie z.B. Kippen, Umstülpen oder Umschwenken der Vorrichtung, miteinander vermischt oder in Kontakt gebracht werden können, ohne daß hierzu ein Öffnen der Vorrichtung erforderlich ist.

Der erfindungsgemäße Testkit wird angewendet in einem Verfahren zur chemischen Analyse von gasförmigen oder in die Gasform überführbaren Probeninhaltsstoffen in einer Vorrichtung der oben geschilderten Art. Bei diesem Verfahren wird die zu analysierende Probe in die Reaktionszone des Behältnisses gegeben, anschließend wird das Behältnis durch eine Verschlußkappe bzw. durch Ansetzen des anderen Behälters mittels des Adapters nach außen gasdicht verschlossen. Hiernach werden die gasförmigen oder gasförmig austreibbaren Bestandteile der Probe in die Detektionszone überführt, wo sie durch Reaktion mit dem festen oder flüssigen Reagenz dessen Veränderung bewirken, welche mit bekannten Meßverfahren ausgewertet wird. Vorzugsweise handelt es sich dabei um optische Veränderungen und Meßverfahren.

Für die Erzeugung der gasförmigen Bestandteile aus der Probe können physikalische, chemische, biochemische

4

oder mikrobiologische Methoden angewandt werden. Als chemische Methoden seien vorzugsweise Ansäuern, Alkalisierung, Oxidation, Reduktion und Derivatisierung genannt.

Die Überführung der gasförmigen Bestandteile aus dem Behälter mit der Reaktionszone in den Behälter mit der Detektionszone kann durch Erzeugung eines höheren Gasdrucks im ersteren Behälter oder durch Erzeugung eines verminderten Gasdrucks im zweiten Behälter beschleunigt werden, so daß über den gemeinsamem Gasraum ein Druckausgleich und somit ein Gastransport aus der Reaktions- in die Detektionszone erfolgt. Ein höhrerer Gasdruck kann z.B. durch eine chemische Reaktion, bei welcher ein Trägergas entsteht, erzeugt werden. Ein verminderter Gasdruck kann z.B. durch Zehrung eines Gases (d.h. durch dessen Absorption) in der Detektionszone bewirkt werden. Die Erzeugung und Überführung der gasförmigen Probenbestandteile kann auch durch Energiezufuhr zur Reaktionszone und/oder durch chemische oder physikalische Reaktionen erfolgen. Als geeignete Mittel der Energiezufuhr kommen u.a. Erwärmung, Bestrahlung, insbesondere mit Ultraviolett oder Mikrowellen, Ultraschallbehandlung oder ein durch die Reaktionszone fließender elektrischer Strom in Frage.

Nach Überführung des Analytgases in die Detektionszone kann es ggf. vorteilhaft sein, daß das zu detektierende Gas dort zunächst nur adsorbiert wird, und die optische Veränderung erst nach Zugabe eines weiteren Reagenzes erfolgt. Dies ist z.B. dann sinnvoll, wenn die Temperaturbelastung infolge der zur Überführung der gasförmigen Bestandteile erforderlichen Erhitzung der Reaktionszone für eine oder mehrere der in der Detektionszone wirksamen Indikatorkomponenten zu hoch ist.

Bei speziellen Anwendungen, z.B. wenn nach Vermischung von Probe und Reagenz eine so spontane Gasfreisetzung erfolgt, daß Gasverluste zu erwarten wären, falls nach Proben- oder Reagenzzugabe die erfindungsgemäße Vorrichtung erst noch mittels Zuschrauben verschlossen werden müßte, gelangt vorteilhafterweise die genannte, partiell abgewandelte Modifikation des erfindungsgemäßen Testkits zum Einsatz. Diese zeichnet sich dadurch aus, daß sie in mindestens einem Teilbereich .- z.B. in der Reaktionszone - in geeigneter Weise zur getrennten Aufnahme von Reagenzien bzw. Proben räumlich unterteilt ist, so daß diese Phasen durch mechanische Manipulation (Kippen, Umschwenken, o.ä.) der geschlossenen Vorrichtung miteinander vermischt oder in Kontakt gebracht werden können, um auf diese Weise Gasverluste zu vermeiden.

Gasförmige Bestandteile können insbesondere Kohlendioxid, Halogene, Cyanwasserstoff, Ammoniak, Stickoxide, Schwefelwasserstoff, Schwefeloxide, Sauerstoff, Mercaptane, Phosphin, Arsenwasserstoff, Stibin, Quecksilber (Dampf), Phenole, Halogenwasserstoff, Kohlenwasserstoffe und/oder organische Säuren sein.

Mit dem beschriebenen Verfahren kann sowohl der in der Probe vorhandene anorganisch gebundene Kohlenstoff (Total Inorganic Carbon TIC) als auch der gesamte gebundene Kohlenstoff (Total Carbon TC) bestimmt werden. Dazu muß er in Kohlendioxid überführt werden. Für die Bestimmung des TIC wird diese Überführung durch einfaches Ansäuern erreicht. Für die Bestimmung des TC wird diese Überführung durch Oxidation erreicht, wobei vorzugsweise der pH-Wert während der Oxidationsvorganges vom alkalischen in den sauren Bereich absinkend gesteuert wird.

Eine bevorzugte Auswertungsmethode für die optischen Veränderungen in der Detektionszone besteht in der Photometrie. Des weiteren ist es im Hinblick auf marktübliche Testkits sinnvoll, zur Durchführung der Analyse benötigte Reagenzien in Form von Fertigtests vorzukonfektionieren und in lagerfähiger Form in den einzelnen verschlossenen Behältern unterzubringen.

Der erfindungsgemäße Testkit kann insbesondere verwandt werden zur chemischen Bestimmung des biologischen Sauerstoffbedarfs (BSB), von gebundenem Kohlenstoff (TC), von anorganisch gebundenem Kohlenstoff (TIC), von organisch gebundenem Kohlenstoff (TOC), von gelöstem organischen Kohlenstoff (DOC), von flüchtigen organisch gebundenem Kohlenstoff (VOC), von ausblasbarem organisch gebundenen Kohlenstoff (POC), von adsorbierbaren organischen Halogenverbindungen (AOX), von gebundenen organischen Halogenverbindungen (TOX), von ausblasbaren organischen Halogenverbindungen (DOX), von gelösten organischen Halogenverbindungen (EOX), von schwerflüchtigen Halogenkohlenwasserstoffen (SHKW), von leichtflüchtigen Halogenkohlenwasserstoffen (LHKW), von gebundenem Stickstoff, von Cyanid, von Schwefel, von Phosphor, von Arsen, von Antimon, von Quecksilber, von Phenolen und von anderen flüchtigen organischen Verbindungen.

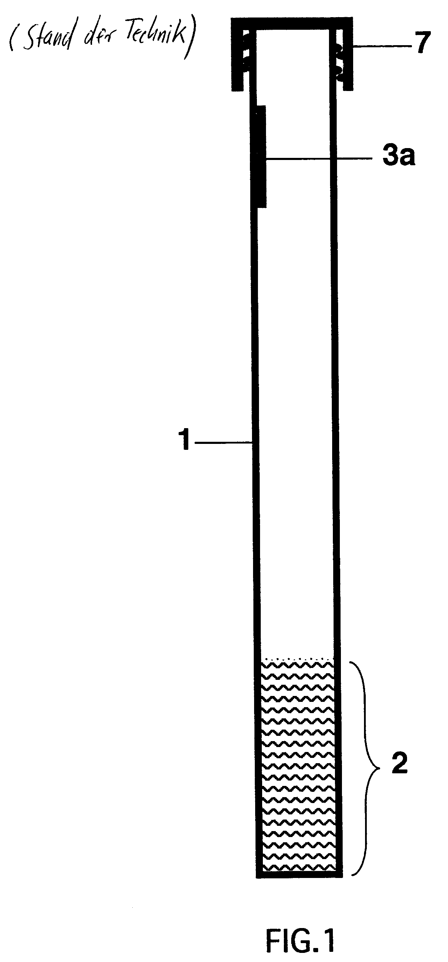

Im folgenden wird die Erfindung anhand der Figuren 1 bis 8 erläutert.

*Figur 1* zeigt eine Vorrichtung nach dem Stand der Technik (vgl. US-A-5,320,807), die im wesentlichen aus nur einem verschließbaren Behälter mit einer erfindungsgemäßen Verschlußvorrichtung 7, vorzugsweise einer Schraubverschlußkappe besteht.

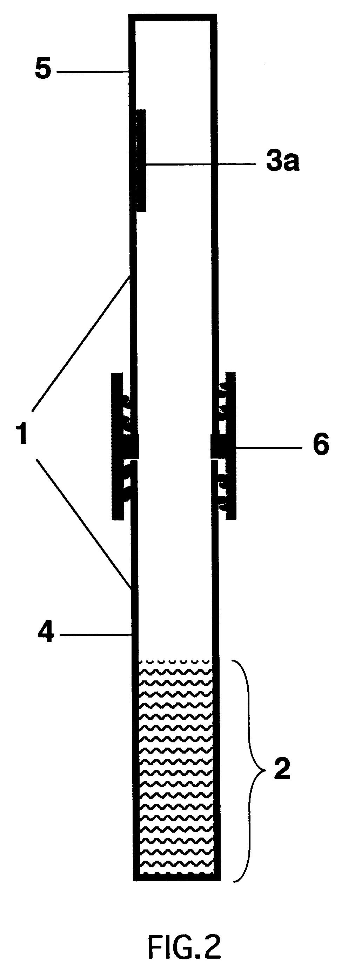

*Figuren 2 bis 8* zeigen Ausführungsformen des erfindungsgemäßen Testkits, bei dem das verschließbare Behältnis aus zwei separaten Behältern 4,5 besteht, die mittels eines Adapters 6 miteinander verbunden sind. Die Reaktionszone 2 dient zur Aufnahme der Probe und ggf. zusätzlicher Reagenzien sowie zur Erzeugung des Analytgases und/oder eines Trägergases, eines erhöhten Gasdruckes und/oder einer Temperaturerhöhung. Die Detektionszone 3a besteht im wesentlichen aus einer auf der Innenseite des Behälters angebrachten sensitiven optischen Festphasendetektionsschicht (z.B. einer Optoden-Membran), die durch Kontakt mit den in der Reaktionszone 2 erzeugten Gasen in selektiver Weise (z.B. durch Absorption, chemische Reaktion oder andere physikalisch-chemische Mechanismen) eine Änderung ihres optischen Verhaltens erfährt, welche durch ein optisches Meßverfahren, wie z.B. Photometrie, Fluorimetrie, Luminometrie, ATR-Photometrie (Abgeschwächte-Total-Reflexions-Photometrie), Reflektometrie oder Bestimmung des Brechungsindex (Refraktometrie) ausgewertet werden kann.

Die Festphasendetektionsschicht kann dabei so ausgebildet sein, daß sie sich nur auf einer Seite der Behälterwandung, an einer festgelegten Stelle der Behälterwandung ("Meßfleck") oder - bei rundem Behälter - auf dem gesamten Behälterumfang befindet. Auch ist es erfindungsgemäß möglich, daß auf den verschiedenen Seiten eines eckigen Behälters oder an verschiedenen festgelegten Stellen der Behälterwandung oder in verschiedenen Höhen eines Rundbehälters in ringförmiger Weise mehrere sensitive optische Festphasendetektionsschichten mit unterschiedlicher Selektivität für verschiedene Gase angebracht werden. Auf diese Weise kann ein Multi-Parameter-Meßsystem zur gleichzeitigen oder sequentiellen Analyse mehrerer gasförmiger Komponenten aus nur einer Probe realisiert werden.

Aufgrund der optischen Meßwerterfassung besteht das erfindungsgemäße Behältnis vorzugsweise aus für diesen Zweck geeignetem optisch transparentem Material.

Bei dem Testkit enthält der Behälter 4 die Reaktionszone 2, und dient deshalb zur Aufnahme der Probe und zur Gaserzeugung aus derselben. Der Behälter 5 enthält die sensitive Festphasendetektionsschicht oder ein flüssiges Reagenz, als Detektionszone 3, 3a, welche durch Absorption und chemische Reaktion der in Behälter 4 erzeugten Gase eine Änderung (z.B. Farbwechsel) erfährt, die mittels geeigneter bekannter Meßmethoden, wie Photometrie, Fluorimetrie, Luminometrie, Refraktometrie, Reflektometrie und ATR-Photometrie ausgewertet werden kann.

Diese Ausführungsformen erlauben es, die Probe in einem der Analyse vorgeschalteten Schritt in dem hierzu abgetrennten Behälter 4 einer Probenvorbereitung zur Erzeugung gasförmig austreibbarer Komponenten zu unterziehen. Die Abtrennung des Behälters 4 zu diesem Zweck ist erforderlich, wenn zur Probenvorbereitung der Einsatz aggressiver Chemikalien unvermeidlich ist (z.B. Säureaufschluß), die das optische Ansprechverhalten der Detektionsphase 3, 3a im Behälter 5 nachteilig beeinträchtigen würden. Hierfür ist es auch denkbar, daß der Adapter einen abdichtenden Verschlußmechanismus enthält, der sich automatisch bei Aufsetzen des Behälters 5 öffnet, oder der von außen geöffnet und verschlossen werden kann. In allen anderen Fällen können die Gaserzeugung aus der Probe und die optische Detektion der erzeugten Gase simultan erfolgen, ohne daß die Behälter 4,5 hierzu voneinander getrennt werden.

Die Aufteilung von Reaktionszone 2 und Detektionszone 3, 3a auf zwei räumlich trennbare Behälter 4,5 ermöglicht es außerdem in vorteilhafter Weise, fertig vorkonfektionierte Chemikalien in den Einzelbehältern getrennt zu lagern und für die Analyse bereitzuhalten, wie dies z.B. für betriebs-analytisch anwendbare chemische Testkits mit photometrischer Testauswertung (sog. "Küvetten-Tests"), erforderlich ist.

Um eine direkte optische Messung der Detektionszone 3,3a - sowie in Ausnahmefällen der Reaktionszone 2 - zu ermöglichen, bestehen die Behälter 4,5 aus optisch transparentem und chemisch inertem Material, vorzugsweise aus optischem Spezialglas (Fiolax- oder AR-Neutralglas).

In einer für die Fertigkonfektionierung bevorzugten Ausführungsform sind die Behälter 4,5 mit Verschlußkappen 7 ausgestattet, deren Verschluß- und Dichtsystem kompatibel ist mit demjenigen des Adapters 6, so daß durch Austausch der Verschlußkappen 7 gegen den Adapter 6 die beiden Behälter 4,5 miteinander verbunden werden, und so die erfindungsgemäße Vorrichtung in einfachster Weise anwendungsbereit gemacht werden kann.

*Figur 2* zeigt eine Ausführung des erfindungsgemäßen Testkits, bei welcher Reaktionszone 2 und Detektionszone 3 des verschließbaren Behältnisses aus zwei separaten Behältern 4,5 bestehen, die über den Adapter 6 miteinander verbunden sind.

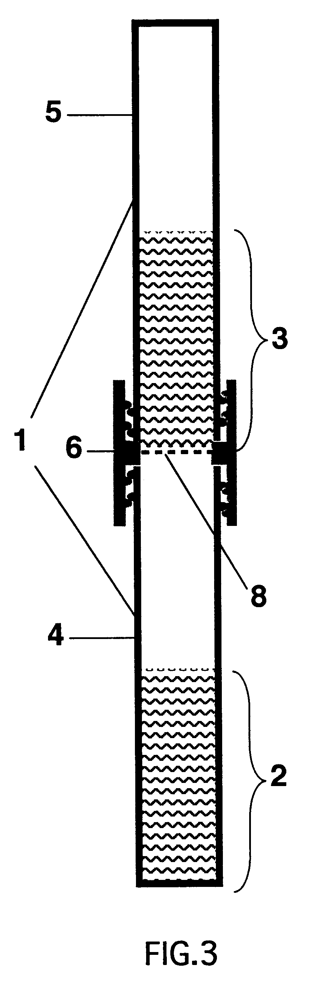

In *Figur 3* enthält der Adapter 6 eine integrierte Membran. Die Membran besitzt eine selektive Durchlässigkeit ausschließlich für gasförmige Komponenten, nicht jedoch für Flüssigkeiten. Das Membranmaterial besteht daher vorzugsweise aus hydrophobem Werkstoff limitierter Porenweite, jedoch sind für die Bestimmung bestimmter gasförmiger Komponenten auch hydrophile Membranmaterialien zulässig. Aufgabe der in den Adapter integrierten Membran ist es, das Detektionsreagenz, im folgenden auch als "Indikator" und/oder "Farbreagenz" bezeichnet, im Behälter 5 vom Inhalt des Reaktionsbehälters 4 zu separieren, so daß keine Vermischung des Indikators oder Farbreagenzes mit der Proben- und/oder Reaktionsflüssigkeit erfolgen kann.

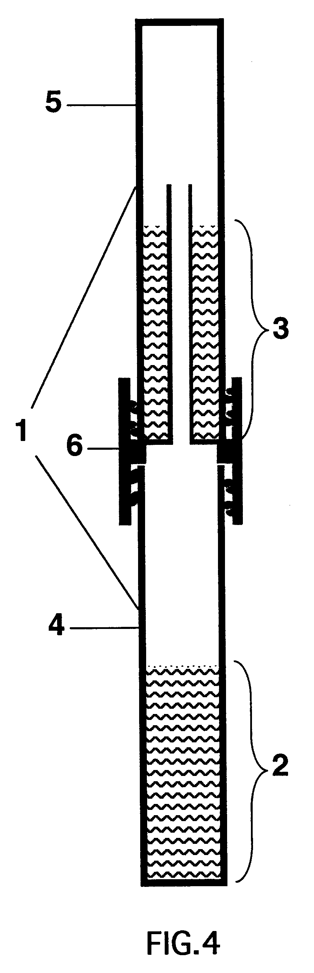

In *Figur 4* ist anstelle einer Membran ein Steigrohr in den Adapter 6 integriert. Dies ermöglicht die gleiche funktionelle Anwendbarkeit der Vorrichtung wie in der Ausführung nach Figur 3.

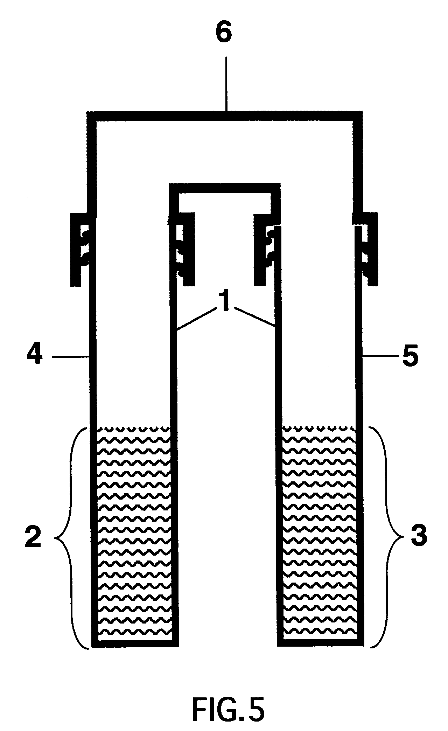

In *Figur 5* besteht der Adapter 6 aus einer U-Rohrverbindung. Diese Ausführungsform entspricht in ihren Anwendungsmöglichkeiten denjenigen der Figur 4.

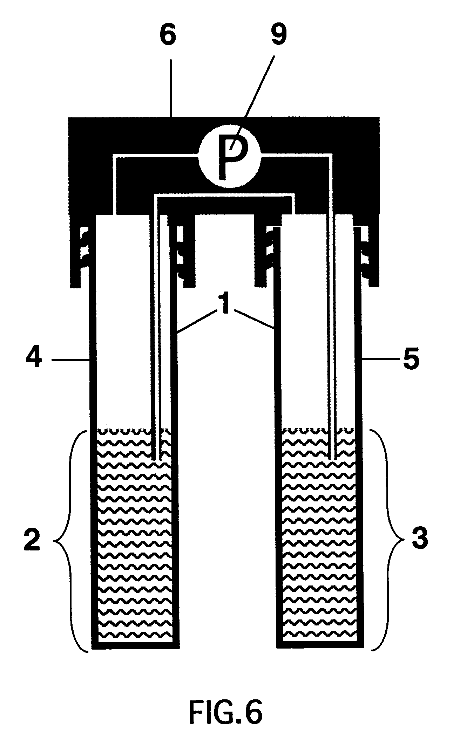

*Figur 6* zeigt eine zur universellen Anwendbarkeit leistungsgesteigerte spezielle Ausführungsform der Vorrichtung nach Abbildung 5. Hierbei wird der Gasaustausch bzw. Gastransfer zwischen der Reaktionszone 2/Behälter 4 und der Detektionszone 3/Behälter 5 mittels einer Gaspumpe 9 dergestalt unterstützt, daß ein geschlossener Gaskreislauf entsteht. Hierdurch wird der Gasaustausch zwischen den Behältern 4,5 infolge des erzwungenen Kreislaufes und des wiederholten Durchströmens der Reaktions- und Detektionszone stark beschleunigt, so daß die in der Detektionszone 3 des Behälters 5 bewirkte Änderung des optischen Verhaltens der sensitiven optischen Festphasendetektionsschicht bzw. des Indikators oder Farbreagenzes bereits nach sehr kurzer Zeit aufgrund vollständiger Überführung der detektierbaren Gase zu einem stabilen Meßsignal führt.

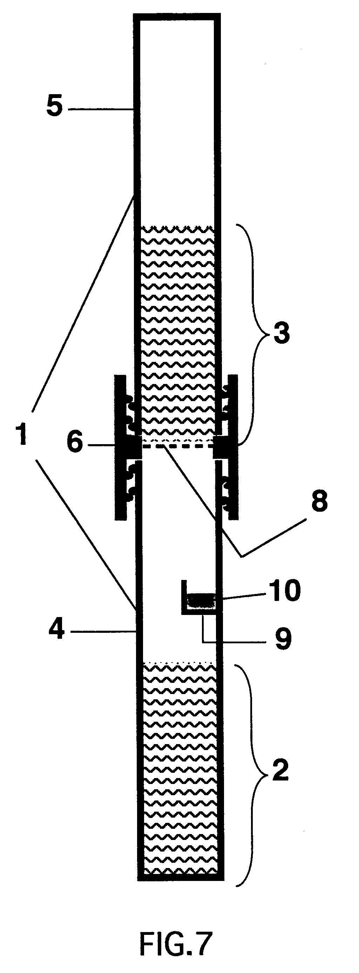

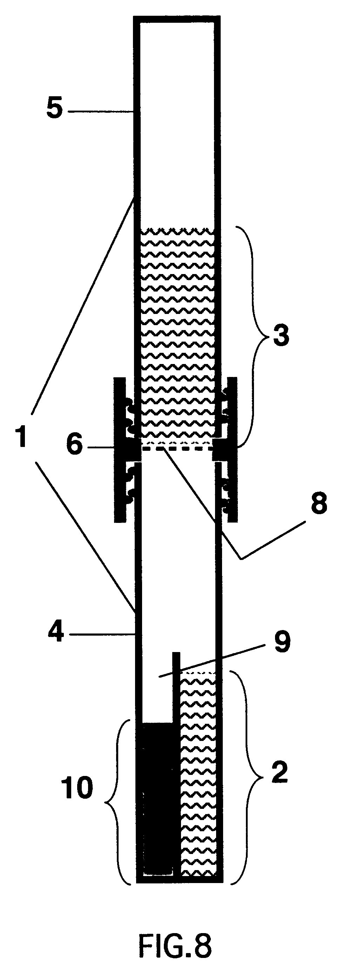

*Figuren 7 und 8* zeigen vorteilhaft modifizierte Ausführungsformen des Testkits nach Abbildung 3, bei denen das Behältnis 1 einen abgegrenzten Teilbereich 9 enthält, so daß zunächst voneinander getrennt zu haltende Proben, Reagenzien oder Phasen 2 und 10 erst nach gasdichtem Verschluß des Behältnisses 1 durch einfache mechanische Manipulation (z.B. Kippen, Umstülpen, Umschwenken) miteinander vermischt oder in Kontakt gebracht werden. Es ist offenkundig, daß die Einführung dieser vorteilhaften Modifikation nicht auf die Ausführungsform nach Abbildung 3 der erfindungsgemäßen Vorrichtung beschränkt bleibt, sondern sinngemäß auch bei den Ausführungsformen gemäß den Abbildungen 2,4,5 und 6 angewendet werden kann.

In den erfindungsgemäßen Ausführungsformen des Testkits gemäß den *Figuren 2-5 und 7 - 8* wird der Gastransfer aus der Reaktionszone 2 in die Detektionszone 3 durch Gasdruckerhöhung - vorzugsweise durch selektiv nur auf die Reaktionszone 2 gerichtete Erwärmung - stark beschleunigt. Zu diesem Zweck ist der die Reaktionszone 2 des Behältnisses umfassende Teil der Vorrichtung bevorzugt derart gestaltet (z.B. zylindrisch, mit geeignetem Durchmesser), daß er in konventionellen, marktgängigen Trockenthermostaten eingesetzt werden kann.

Die in den erfindungsgemäßen Vorrichtungen entsprechend den *Figuren 2-8* verwendeten Festphasendetektionsschichten, Indikatoren und Farbreagenzien können reversibel oder irreversibel mit den in der Reaktionszone 2 erzeugten Gasen reagieren.

Im Fall der irreversiblen Reaktion ist nur eine einmalige Anwendung der Vorrichtung oder des Behälters 5 mit der Detektionszone 3 möglich. Die irreversible Reaktion ermöglicht in bestimmten Anwendungsfällen sogar eine Anreicherung der zu analysierenden Gase, und hiermit eine höhere Nachweisempfindlichkeit.

Festphasendetektionsschichten und/oder Indikatoren mit reversibler Reaktion der erzeugten Gase sind mehrfach wiederverwendbar, und können z.B. durch Spülung mit Inertgas - oder im einfachsten Fall durch einfache Raumluft-Exposition - regeneriert und für eine Wiederverwendung aufbereitet werden.

Die Ausführungsformen des erfindungsgemäßen Testkits gemäß den *Figuren 3-8* eignen sich darüber hinaus zur Überführung destillativ abtrennbarer Flüssigkeiten aus der Reaktionszone 2 in die Detektionszone 3, indem zwischen Reaktions- und Detektionszone ein ausreichend großer Temperaturgradient erzeugt wird (z.B. durch selektive Beheizung nur der Reaktionszone in einem marktgängigen Trockenthermostaten), so daß die in der Reaktionszone erzeugten Dampfmoleküle in der kühlen Detektionszone kondensieren und dort Veränderungen der sensitiven Festphasendetektionsschicht oder des Indikators bewirken können.

Die Anwendungsmöglichkeiten des erfindungsgemäßen Testkits und ihrer speziellen Ausführungsformen gemäß den Abbildungen 2-8 seien anhand folgender Beispiele aufgezeigt.

**Beispiel 1:**

Dieses Beispiel betrifft ein Analyseverfahren in einer Vorrichtung nach dem Stand der Technik, wie sie z.B. hergestellt ist in Abb. 1.

Bestimmung des Carbonatgehaltes einer Wasserprobe:

Die sensitive optische Festphasendetektionsschicht der Detektionszone (3) ist eine Kohlendioxid-sensitive Farbmembran, deren Herstellung prinzipiell nach dem Stand der Technik erfolgt (siehe z.B. A.Mills, Q.Chang, N.McMurray: Anal.Chem. 1992, 64, 1383-1389).

In der Reaktionszone (2) des Behältnisses (1) wird ein vorzugsweise festes, carbonatfreies, gut wasserlösliches Säuerungsmittel (z.B. Citronensäure) vorgelegt. Nach Zusatz einer carbonathaltigen Wasserprobe wird das Behältnis sofort mit der Schraubverschlußkappe (7) dichtend verschlossen. Durch Kontakt mit der Wasserprobe wird das Säuerungsmittel aufgelöst, wodurch der pH-Wert der Wasserprobe soweit abgesenkt wird, daß das Carbonatgleichgewicht vollständig in Richtung des freien Kohlendioxids verschoben wird. Durch die der Konzentration an gelöstem Kohlendioxid entsprechende Lage des Kohlendioxidgleichgewichtes (Verteilung zwischen flüssiger Phase und Gasraum) verfärbt sich die Kohlendioxid-sensitive Membran von blau nach gelb. Dieser Vorgang kann durch gezielte Erwärmung der Reaktionszone (2) - z.B. im Trockenthermostat - erheblich beschleunigt werden. Für die quantitative Bestimmung des Carbonatgehaltes der Wasserprobe kann die Zunahme der Gelbfärbung bei 420 nm oder die Abnahme der Blaufärbung bei 600 nm photometrisch ausgewertet, und über eine entsprechende Kalibration zur Quantifizierung der Carbonat-Konzentration genutzt werden.

**Beispiel 2:**

Bestimmung des Gesamtkohlenstoffs (TC), des anorganisch gebundenen Kohlenstoffs (TIC) und des organisch gebundenen Kohlenstoffs (TOC) von Abwasserproben mit dem erfindungsgemäßen Testkit nach Abbildung 3.

a) Arbeitsgang zur Bestimmung des *Gesamtkohlenstoffs (TC)* mit der erfindungsgemäßen Vorrichtung nach Abbildung 3:

- Schraubverschlußkappe (7) von Behälter (5) mit 2,0 ml vorkonfektionierter Indikatorlösung entfernen

- Adapter (6) mit integrierter Membran auf Behälter (5) aufschrauben

7

- Schraubverschlußkappe (7) von Behälter (4) mit 1,0 ml carbonatfreier Pufferlösung entfernen

- definierte Menge eines festen, pulverförmigen Oxidationsmittels mit einer Dosierhilfe in den Behälter (4) dosieren

- 2,0 ml Wasserprobe in den Behälter (4) pipettieren

- Behälter (4) mit der Kombination aus Adapter (6) und Behälter (5) fest verschließend

- Vorrichtung mit Behälter (4) so tief in einen Trockenthermostaten einsetzen, daß im wesentlichen nur die Reaktionszone (2) erwärmt wird, und 2 Stunden lang auf 100°C erhitzen

- nach Abkühlung auf Raumtemperatur die Vorrichtung mit dem Behälter (5) in den Küvettenschacht eines Photometers einsetzen, und die Verfärbung des Indikators bei einer Meßwellenlänge von 600 nm auswerten

- Berechnung des Gehaltes an Gesamtkohlenstoff nach Kalibration und linearer Regression:
$$C(TC)_{mg/l} = \text{Extinktion}_{600\ nm} * \text{Faktor}_{TC} + \text{Konstante}_{TC}$$

b) Arbeitsgang zur Bestimmung des *anorganisch gebundenen Kohlenstoffs (TIC)* mit dem erfindungsgemäßen Testkit nach Abbildung 3:

- Schraubverschlußkappe (7) von Behälter (5) mit 2,0 ml vorkonfektionierter Indikatorlösung entfernen

- Adapter (6) mit integrierter Membran auf Behälter (5) aufschrauben

- Schraubverschlußkappe (7) von Behälter (4) mit 1,0 ml vorkonfektionierter Säuremischung entfernen

- 2,0 ml Wasserprobe in den Behälter (4) pipettieren und Behälter (4) mit der Kombination aus Adapter (6) und Behälter (5) fest verschließen

- Vorrichtung mit Behälter (4) in einen Trockenthermostaten einsetzen und 2 Stunden lang auf 100°C erhitzen

- nach Abkühlung auf Raumtemperatur die Vorrichtung mit dem Behälter (5) in den Küvettenschacht eines Photometers einsetzen, und die Verfärbung des Indikators bei einer Meßwellenlänge von 600 nm auswerten

- Berechnung des Gehaltes an anorganisch gebundenem Kohlenstoff nach Kalibration und linearer Regression:

$$C(TIC)_{mg/l} = \text{Extinktion}_{600\ nm} * \text{Faktor}_{TIC} + \text{Konstante}_{TIC}$$

c) Berechnung des *organisch gebundenen Kohlenstoffs (TOC)* aus der Differenz der vorgehend beschriebenen Analysen nach a) und b):

$$TOC_{mg/l} = TC_{mg/l} - TIC_{mg/l}$$

Nachstehende Tabelle verdeutlicht die gute Korrelation des erfindungsgemäßen Verfahrens zur Bestimmung von Gesamtkohlenstoff (TC), anorganisch gebundenen Kohlenstoff (TIC) und organisch gebundenem Kohlenstoff (TOC) mit einer nach dem Stand der Technik arbeitenden Vergleichsmethode (halbautomatischer TOC Analyzer „Shimadzu TOC-5050"; Verfahren nach DEV/DIN 38409-H3 und US-Standard Methods, Chapter 505):

|  |  | Erfindungsgemäßes Verfahren (Vorrichtung nach Abb.3) | Shimadzu TOC-5050 |
|---|---|---|---|
| Probe 1 (kommunales Abwasser, Kläranlagenablauf) | TC | 23,88 mg/l | 24,48 mg/l |
|  | TIC | 15,80 mg/l | 15,51 mg/l |
|  | TOC | 8,08 mg/l | 8,97 mg/l |
| Probe 2 (kommunales Abwasser, Kläranlagenzulauf) | TC | 143,57 mg/l | 139,30 mg/l |
|  | TIC | 58,38 mg/l | 55,94 mg/l |
|  | TOC | 85,19 mg/l | 83,36 mg/l |
| Probe 3 (Industrieabwasser) | TC | 20,40 mg/l | 19,74 mg/l |
|  | TIC | 9,85 mg/l | 9,77 mg/l |
|  | TOC | 10,55 mg/l | 9,97 mg/l |

Das Beispiel zeigt, daß mit dem erfindungsgemäßen Testkit der abwasseranalytisch äußerst bedeutsame Parameter TOC kostengünstig und in einfacher Weise - d.h. durch wenige, unkomplizierte Handhabungsschritte - bestimmbar ist. Dies ist angesichts der hohen Kosten (mehr als 50.000 DM) von nach dem bisherigen Stand der Technik arbeitenden TOC-Analysengeräten von erheblicher Bedeutung, da das erfindungsgemäße Verfahren in Verbindung mit dem erfindungsgemäßen Testkit erstmals eine einfache und kostengünstige TOC-Analytik im Rahmen einer betriebsanalytisch üblichen Grundausstattung auf der Basis Photometer, Trockenthermostat und TOC-Testkit ermöglicht.

**Beispiel 3:**

Bestimmung von Ammonium-Stickstoff ($NH_4$-N) aus stark belasteten und verfärbten Abwasserproben mit dem erfindungsgemäßen Testkit nach Abbildung 6.

An den Adapter (6) mit der integrierten Gaspumpe (9) wird der Behälter (5) mit einer vorkonfektionierten Indikatorlösung angeschraubt. Zur Bestimmung des Ammonium-Stickstoffs besteht die Indikatorlösung aus einem stark alkalisch gepufferten Salicylatreagenz, welches zusätzlich ein geeignetes Chlorierungsmittel, wie z.B. Hypochlorit, und als Katalysator Natriumnitroprussid enthält (Indophenolblau-Methode nach Berthelot). In der Reaktionszone (2) des Behälters (4) wird ein vorzugsweise festes, ammoniumfreies Alkalisierungsmittel (z.B. 150 mg Lithiumhydroxid-Monohydrat) vorgelegt. Nach Zusatz einer ammoniumhaltigen Wasserprobe wird der Behälter (4) sofort ebenfalls an den Adapter (6) angeschlossen. Durch das Einschalten der Gaspumpe ("P") werden die Flüssigkeiten in den Behältern (4) und (5) kräftig durchmischt, wodurch sich das Alkalisierungsmittel im Behälter (4) in der Wasserprobe auflöst und deren pH-Wert soweit anhebt, daß das Ammonium/Ammoniak-Gleichgewicht vollständig in Richtung des freien Ammoniakgases verschoben wird. Durch den mittels der Gaspumpe ständig umgewälzten Gasraum der Vorrichtung wird das gebildete Ammoniakgas aus der Reaktionszone (2) ausgestrippt und in der Indikatorlösung des Behälters (5) absorbiert.

Die sofort auftretende und sich allmählich verstärkende Grünblau-Färbung des Indikators ermöglicht eine optische Überwachung des Stripp-Prozesses. Nach 15 Minuten Strippzeit, innerhalb derer sich die Farbtiefe bis zum vollständigen Umsatz des übergetriebenen Ammoniaks weiter vertieft, wird der Behälter (5) von dem Adapter (6) gelöst, mit einer Schraubverschlußkappe (7) verschlossen, und die aufgetretene Verfärbung des Reagenzes bei 695 nm photometrisch ausgewertet. Die Quantifizierung der Ammonium-Stickstoffkonzentration erfolgt über eine entsprechende Kalibration des Meßsystems.

Übliche, an Standards verifizierte Wiederfindungsraten dieses Verfahrens liegen bei 97-100 % der Theorie.

Das Beispiel zeigt, daß durch den Einsatz des erfindungsgemäßen Testkits nach Abbildung 6 in schneller und einfacher Weise eine Abtrennung und Bestimmung von Ammonium-Stickstoff aus einer Wasserprobe möglich ist. Dies ist von erheblicher Bedeutung insbesondere für die Bestimmung des organischen Stickstoffs nach dem Kjeldahl-Verfahren (DEV, H 11), der Bestimmung des Nitrat-Stickstoffs nach der Devarda-Methode (U.S. Standard Methods, Chapter 418E), sowie der Bestimmung des Gesamt-Stickstoffs mit der kombinierten Devarda/Kjeldahl-Methode (DEV/DIN 38409, H 28). Bei diesen Methoden kann durch Einsatz des erfindungsgemäßen Testkits nach Abbildung 6 auf die bisher erforderliche aufwendige Wasserdampfdestillation verzichtet werden. Durch die direkte Überführung des Analytgases (Ammoniak) in eine Meßvorlage (z.B. Rundküvette, identisch mit Behälter (5)) wird die Gesamtanalysendauer mindestens um die Dauer des bisher erforderlichen Destillationsschrittes verkürzt.

Die beispielhaft aufgeführten Anwendungsmöglichkeiten des erfindungsgemäßen Testkits und seiner speziellen Ausführungsformen, sowie der hieraus abzuleitenden analytischen Verfahren verdeutlichen die Bedeutung der vorliegenden Erfindung insbesondere im Hinblick auf ihre Einbindung in bereits bestehende modulare Analysensysteme der Betriebsanalytik (Photometer und Trockenthermostat).

**Patentansprüche**

1. Testkit zur chemischen Analyse von gasförmigen oder in die Gasform überführbaren Probeninhaltsstoffen,

   a) mit zwei separaten einzelnen Behältern (4,5), von denen der erste (4) der Aufnahme der Probe und der zweite (5) der Aufnahme der aus der Probe freigesetzten Gase dient, wobei
   b) der zweite Behälter (5) ein gassensitives Reagenz enthält, das durch Kontakt mit dem in dem ersten Behälter (4) erzeugten Gas eine optische Änderung erfährt,
   c) der zweite Behälter (5) so ausgestaltet ist, daß er in ein optisches Meßgerät als Meßvorlage einsetzbar ist,
   d) die Behälter (4,5) jeweils mit einem Verschluß (7) versehen sind, und
   e) der Testkit weiterhin einen Adapter (6) umfaßt, durch den die Behälter (4,5) nach Abnahme der Verschlüsse (7) gasdicht miteinander verbindbar sind.

2. Testkit nach Anspruch 1,
   dadurch gekennzeichnet, daß der Adapter mit einer semipermeablen Membran (8) versehen ist, die ausschließlich für Gase durchlässig ist.

3. Testkit nach Anspruch 2,
   dadurch gekennzeichnet, daß die Membran (8) aus hydrophobem Material besteht.

4. Testkit nach einem der Ansprüche 1 bis 3,
   dadurch gekennzeichnet, daß in den Adapter eine Gaspumpe (9) integriert ist.

5. Testkit nach einem der Ansprüche 1 bis 4,
   dadurch gekennzeichnet, daß die Behälter (4,5) mit einem Gewinde versehen sind, so daß die Verschlüsse (7) oder der Adapter (6) aufschraubbar sind.

6. Testkit nach einem der Ansprüche 1 bis 5,
   dadurch gekennzeichnet, daß der erste Behälter (4) zur Erzeugung gasförmiger Bestandteile aus der Probe Stoffe in vorkonfektionierter und lagerfähiger Form enthält.

7. Testkit nach einem der Ansprüche 1 bis 6,
   dadurch gekennzeichnet, daß der zweite Behälter (5) das gassensitive Reagenz in fester oder flüssiger Form enthält.

8. Testkit nach einem der Ansprüche 1 bis 7,
   dadurch gekennzeichnet, daß der zweite Behälter (5) das gassensitive Reagenz in vorkonfektionierter und lagerfähiger Form enthält.

9. Testkit nach einem der Ansprüche 1 bis 8,
   dadurch gekennzeichnet, daß das gassensitive Reagenz einen gelösten Indikator oder ein Farbreagenz enthält.

10. Verwendung des Testkits nach einem der Ansprüche 1 bis 9 zur chemischen Bestimmung des biologischen Sauerstoffbedarfs (BSB), von gebundenem Kohlenstoff (TC), von anorganisch gebundenem Kohlenstoff (TIC), von organisch gebundenem Kohlenstoff (TOC), von gelöstem organischen Kohlenstoff (DOC), von flüchtigem organisch gebundenen Kohlenstoff (VOC), von ausblasbarem organisch gebundenen Kohlenstoff (POC), von adsorbierbaren organischen Halogenverbindungen (AOX), von gebundenen organischen Halogenverbindungen (TOX), von ausblasbaren organischen Halogenverbindungen (POX), von gelösten organischen Halogenverbindungen (DOX), von extrahierbaren organischen Halogenverbindungen (EOX), von schwerflüchtigen Halogenkohlenwasserstoffen (SHKW), von leichtflüchtigen Halogenkohlenwasserstoffen (LHKW), von gebundenem Stickstoff, von Cyanid, von Schwefel, von Phosphor, von Arsen, von Antimon, von Quecksilber, von Phenolen und von anderen flüchtigen organischen Verbindungen.

**Claims**

1. Test kit for the chemical analysis of gaseous contents of samples, or of those contents which can be converted into gaseous form,

   a) with two separate individual containers (4, 5), of which the first container (4) is used for receiving the sample, and the second container (5) is used for receiving the gases released from the sample, where
   b) the second container (5) contains a gas-sensitive reagent which undergoes an optical change upon contact with the gas generated in the first container (4),
   c) the second container (5) is designed such that it can be fitted as measurement vessel into an optical measuring unit,
   d) the containers (4, 5) are each provided with a closure member (7), and
   e) the test kit furthermore comprises an adapter (6) by means of which the containers (4, 5) can be connected to each other in a gastight manner after the closure members (7) have been removed.

2. Test kit according to Claim 1, characterized in that the adapter is provided with a semipermeable membrane (8), which is permeable exclusively to gases.

3. Test kit according to Claim 2, characterized in that the membrane (8) consists of hydrophobic material.

4. Test kit according to one of Claims 1 to 3, characterized in that a gas pump (9) is incorporated into the adapter.

5. Test kit according to one of Claims 1 to 4, characterized in that the containers (4, 5) are provided with a thread, so that the closure members (7) or the adapter (6) can be screwed on.

6. Test kit according to one of Claims 1 to 5, characterized in that the first container (4) for generating gaseous constituents from the sample contains materials in made-up and storable form.

7. Test kit according to one of Claims 1 to 6, characterized in that the second container (5) contains the gas-sensitive reagent in solid or liquid form.

8. Test kit according to one of Claims 1 to 7, characterized in that the second container (5) contains the gas-sensitive reagent in made-up and storable form.

9. Test kit according to one of Claims 1 to 8, characterized in that the gas-sensitive reagent contains a dissolved indicator or a colour reagent.

10. Use of the test kit according to one of Claims 1 to 9 for the chemical determination of biological oxygen demand (BOD), total carbon (TC), total inorganic carbon (TIC), total organic carbon (TOC), dissolved organic carbon (DOC), volatile organic carbon (VOC), particulate organic carbon (POC), adsorbable organic halogen compounds (AOX), total organic halogen compounds (TOX), particulate organic halogen compounds (POX), dissolved organic halogen compounds (DOX), extractable organic halogen compounds (EOX), non-volatile halocarbons (NVHC), readily volatile halocarbons (RVHC), total nitrogen, cyanide, sulphur, phosphorus, arsenic, antimony, mercury, phenols, and other volatile organic compounds.

**Revendications**

1. Test kit destiné à l'analyse chimique de substances gazeuses contenues dans des échantillons ou de telles substances contenues dans des échantillons, qui sont convertibles en état gazeux,

   a) avec deux récipients individuels séparés (4, 5) dont le premier (4) sert à recevoir l'échantillon et le deuxième (5) sert à recevoir les gaz dégagés de l'échantillon,
   b) le deuxième récipient (5) contenant un réactif sensitif au gaz, qui connaît un changement optique en raison du contact avec le gaz produit dans le premier récipient (4),
   c) le deuxième récipient (5) étant réalisé de telle manière à ce qu'il puisse être placé en tant que récipient de mesure dans un appareil de mesure optique,
   d) les récipients (4, 5) étant respectivement pourvus d'une fermeture (7), et
   e) le test kit comprenant en sus un adaptateur (6) par l'intermédiaire duquel les récipients (4, 5) peuvent être reliés l'un à l'autre d'une manière étanche aux gaz après avoir ôté les fermetures (7).

2. Test kit selon la revendication 1,
   caractérisé par le fait que l'adaptateur est pourvu d'une membrane (8) semiperméable qui est perméable exclusivement aux gaz.

3. Test kit selon la revendication 2,
   caractérisé par le fait que la membrane (8) se compose de matière hydrophobe.

4. Test kit selon l'une des revendications 1 à 3,
   caractérisé par le fait qu'une pompe à gaz (9) est intégrée dans l'adaptateur.

5. Test kit selon l'une des revendications 1 à 4,
   caractérisé par le fait que les récipients (4, 5) sont pourvus d'un filet de sorte que les fermetures (7) ou l'adaptateur (6) peuvent être vissés.

6. Test kit selon l'une des revendications 1 à 5,
   caractérisé par le fait que le premier récipient (4) servant à produire des composants gazeux à partir de l'échantillon, contient des matières en forme préconfectionnée et stockable.

7. Test kit selon l'une des revendications 1 à 6,
   caractérisé par le fait que le deuxième récipient (5) contient le réactif sensitif au gaz, en forme solide ou liquide.

8. Test kit selon l'une des revendications 1 à 7,
   caractérisé par le fait que le deuxième récipient (5) contient le réactif sensitif au gaz, en forme préconfectionnée et stockable.

9. Test kit selon l'une des revendications 1 à 8,
   caractérisé par le fait que le réactif sensitif au gaz contient un indicateur dissous ou un réactif coloré.

10. Utilisation du test kit selon l'une des revendications 1 à 9, pour la détermination chimique de la demande d'oxygène biologique (D.B.O.), de carbone combiné (TC), de carbone combiné inorganiquement (TIC), de carbone combiné organiquement (TOC), de carbone organique dissous (DOC), de carbone volatil combiné organiquement (VOC), de carbone combiné organiquement qui peut être éliminé en soufflant (POC), de combinaisons d'halogène organiques adsorbables (AOX), de combinaisons d'halogène organiques combinées (TOX), de combinaisons d'halogène organiques qui peut être éliminées en soufflant (POX), de combinaisons d'halogène organiques dissoutes (DOX), de combinaisons organiques extractibles (EOX), d'hydrocarbures halogénés difficilement volatils (SHKW), d'hydrocarbures halogénés facilement volatils (LHKW), d'azote combiné, de cyanure, de soufre, de phosphore, d'arsenic, d'antimoine, de mercure, de phénols et d'autres combinaisons organiques volatiles.

( Stand der Technik)

**FIG.1**

FIG.2

FIG.3

FIG.4

FIG.5

FIG.6

**FIG.7**

FIG.8